# EUROPÄISCHE PATENTANMELDUNG

(11) **EP 0 768 065 A1**
(43) Veröffentlichungstag der Anmeldung: **16.04.1997**
(21) Anmeldenummer: 96115753.4
(22) Anmeldetag: 02.10.1996
(51) Int. Cl.: A61C 7/00, A61B 17/80

(54) **Zugapparat zur Korrektur eines Oberkiefers**

(30) Priorität: 14.10.1995 DE 19538323
(71) Anmelder: Müller, Paul A., Dr. med., 8034 Zürich (CH)
(72) Erfinder: Müller, Paul A., Dr. med., 8034 Zürich (CH)
(74) Vertreter: Weiss, Peter, Dr. rer. nat.

(57) **Zusammenfassung**

Bei einem Zugapparat zur Korrektur eines Oberkiefers (19) an einem Schädel (20), beispielsweise zur Beseitigung eines Überbisses, Kreuzbisses oder Gaumenspaltes, soll ein Grundkörper (1) mit Elementen (4, 18, 22) zum Festlegen am Oberkiefer (19) versehen und in Korrekturrichtung (x₁) bewegbar sein.

## Beschreibung

Die Erfindung betrifft einen Zugapparat zur Korrektur eines Oberkiefers an einem Schädel, beispielsweise zur Beseitigung eines Überbisses, Kreuzbisses oder Gaumenspaltes.

Korrekturen am Oberkiefer, insbesondere zur Erzielung einer Okklusion, werden heute meist durch eine Operation nach Le Fort durchgeführt. Diese Operation dauert ca. zwei bis drei Stunden und kann nur in stationärer Behandlung durchgeführt werden. Eine Hospitalisation ist notwendig.

Bei der Operation nach Le Fort wird der Oberkiefer gänzlich vom Schädel abgetrennt und auch die Nasenschleimhaut abgetrennt, der Oberkiefer nach vorne versetzt und meist mittels Titanplättchen neu fixiert.

Abgesehen von der erforderlichen aufwendigen Operation stellt sich oft der Nachteil heraus, dass der Kiefer trotz der Fixierungen zurückfällt und somit eine erneute Operation notwendig wird.

Der vorliegenden Erfindung liegt die Aufgabe zugrunde, einen oben erwähnten Zugapparat zu entwickeln, bei dem eine ambulante Behandlung ausreicht und die Gefahr eines Zurückfallens des Kiefers nicht gegeben ist.

Zur Lösung dieser Aufgabe führt, dass ein Grundkörper mit Elementen zum Festlegen am Oberkiefer versehen und in Korrekturrichtung bewegbar ist.

Das bedeutet, dass kein Abtrennen des Oberkiefers mehr erforderlich ist, sondern dass mittels des Zugapparates im Laufe der Zeit eine Korrektur des Oberkiefers möglich wird. Da diese Korrektur langsam vonstatten geht, wird der Oberkiefer quasi an seine neuen Stellungen "gewöhnt", so dass die Gefahr eines nachträglichen Zurückfallens des Oberkiefers völlig beseitigt ist. Ein Einsetzen des Zugapparates geschieht in einer ambulanten Operation, die nicht länger als eine halbe bis eine Stunde dauert.

Ein Einstellen des Zugapparates bzw. auch ein Nachstellen kann von dem Patienten selbst vorgenommen werden, so dass ein erneuter Klinikbesuch nicht notwendig wird.

In der Regel wird der erfindungsgemässe Zugapparat zur Korrektur des Oberkiefers nach vorne eingesetzt. Vom Erfindungsgedanken soll jedoch auch umfasst sein, dass eine Korrektur des Oberkiefers nach hinten möglich ist, wobei dann entsprechende Befestigungsmöglichkeiten gewählt werden müssen.

Ferner kann ein Zugapparat auf einer Seite oder aber jeweils ein Zugapparat auf jeweils einer Seite des Oberkiefers angesetzt werden, so dass ein gleichmässiges Verschieben des Oberkiefers nach vorne bzw. in Ausnahmefällen nach hinten möglich wird.

In einem bevorzugten Ausführungsbeispiel ist zur Verankerung des Grundkörpers an einem Schaft ein Haken zum Hintergreifen des Oberkieferknochens oberhalb des letzten Backenzahnes vorgesehen. Dabei ist bevorzugt der Schaft über einen Bogen mit dem Haken einstückig verbunden, wobei Bogen und Haken einen Hinterschnitt ausbilden, der den Backenknochen in Gebrauchslage aufnimmt. Hierdurch wird ein guter Zug auf den Backenknochen nach vorne ausgeübt.

Denkbar ist auch die Festlegung des Grundkörpers an dem Oberkiefer durch entsprechende Befestigungselemente. Bevorzugt werden dabei Schrauben, die entsprechende Querbohrungen durchsetzen und in den Oberkieferknochen eingeschraubt werden. Im bevorzugten Ausführungsbeispiel werden allerdings beide Festlegungen vorgenommen, d.h. der Grundkörper wird mittels den Schrauben am Oberkiefer fixiert, wobei gleichzeitig der Haken den Oberkieferknochen umgreift. Diese Halterung gewährleistet eine sichere Festlegung des Zugapparates und bewirkt gleichzeitig den günstigsten Angriff an den Oberkieferknochen, um ihn nach vorne zu ziehen.

Zur Lageveränderung des Grundkörpers ist in dem Grundkörper bzw. dem Schaft eine Durchgangsbohrung vorgesehen, in welcher ein Schieber eingesetzt ist, der von einem Betätigungselement bewegt werden kann. Dabei ist dieser Schieber entgegen der Korrekturrichtung verschiebbar. Das bedeutet, dass in dem Augenblick, wenn der Schieber bewegt wird, sich der Grundkörper bzw. Schaft in entgegengesetzter Richtung bewegt. Hierzu ist es allerdings notwendig, dass der Schieber möglichst ortsfest verbleibt, weshalb ihm ein Fähnchen zugeordnet ist, das aus dem Schaft herausragt. An diesem Fähnchen ist eine Halterung festgelegt, welche das Fähnchen mit einem anderen Teil des Oberkiefers oder des Schädels verbindet. Hierdurch findet eine indirekte Festlegung des Fähnchens bzw. des Schiebers statt, so dass bei einem Betätigen des Betätigungselementes nicht das Fähnchen bzw. der Schieber sondern der Grundkörper und damit der mit dem Grundkörper verbundene Oberkiefer bewegt wird.

Der Einfachheit halber besteht die Halterung aus einem Draht, der einends mit dem Fähnchen und andernends mit einem Titanplättchen od. dgl. verbunden ist. Letzteres ist dann beispielsweise über Schrauben an einem anderen Teil des Schädels bzw. Oberkiefers festgelegt.

Das Betätigungselement soll im vorliegenden Ausführungsbeispiel um seine Längsachse drehbar sein, weshalb es einen Gewindeabschnitt aufweist, der in der Durchgangsbohrung dreht. Der Gewindeabschnitt besitzt ein entsprechendes Aussengewinde, die Durchgangsbohrung ein entsprechendes Innengewinde. Allerdings dreht das Betätigungselement gegenüber dem Schieber frei, so dass es den Schieber in der Durchgangsbohrung wegdrücken kann bzw. den Grundkörper gegenüber dem Schieber zieht.

Zur besseren Zentrierung bzw. Halterung des Betätigungselementes gegenüber dem Schieber weist das Betätigungselement einerseits des Gewindeabschnittes einen Zapfen auf, der in eine Sacklochbohrung stirnwärts in den Schieber eingreift. Auch dieser Zapfen dreht gegenüber dem Schieber frei.

Andernseits ist an dem Gewindeabschnitt ein Stangenabschnitt festgelegt, der beispielsweise einen Sechskantkopf besitzt. Dieser Sechskantkopf kann von einem entsprechenden Werkzeug angegriffen und durch Drehen des Werkzeuges das Betätigungselement um seine Längsachse gedreht werden.

Es versteht sich von selbst, dass die beschriebene Betätigungsanordnung aus Schieber und Betätigungselement nur beispielhaft ist. Im Rahmen der Erfindung liegen hier auch andere Möglichkeiten der Bewegung des Grundkörpers in Korrekturrichtung.

Weitere Vorteile, Merkmale und Einzelheiten der Erfindung ergeben sich aus der nachfolgenden Beschreibung bevorzugter Ausführungsbeispiele sowie anhand der Zeichnung; diese zeigt in
Figur 1 eine Draufsicht auf einen erfindungsgemässen Zugapparat;
Figur 2 eine teilweise geschnitten dargestellte Seitenansicht des Zugapparates gemäss Figur 1;
Figur 3 eine Seitenansicht eines Teils eines menschlichen Schädels mit angesetztem Zugapparat;
Figur 4 eine Draufsicht auf einen Teil des menschlichen Schädels gemäss Figur 3 im Bereich des Oberkiefers.

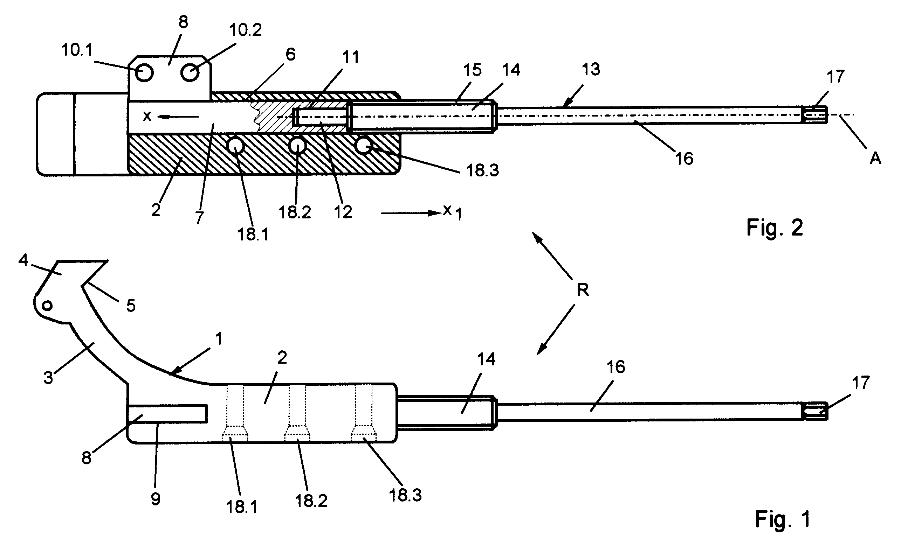

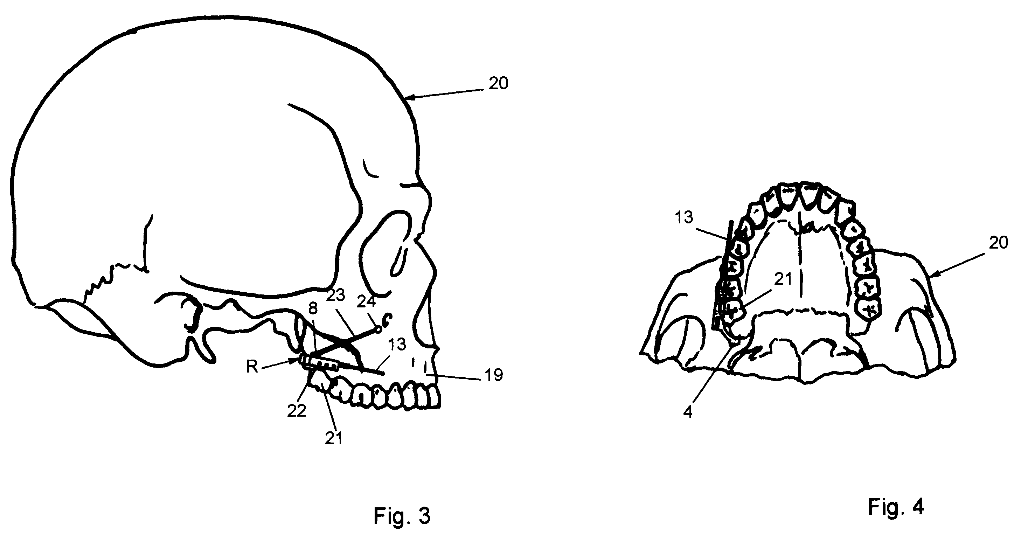

Ein erfindungsgemässer Zugapparat R, dessen Funktion im Zusammenhang mit den Figuren 3 und 4 beschrieben wird, weist gemäss Figur 1 einen Grundkörper 1 auf. Dieser Grundkörper 1 besteht im wesentlichen aus einem Schaft 2, an den sich einstückig über einen Bogen 3 ein Haken 4 anschliesst. Dieser Haken 4 bildet zusammen mit dem Bogen 3 einen Hinterschnitt 5, mit dem der Zugapparat R, wie später beschrieben, einen Oberkiefer (Maxilla) einseitig hintergreift.

In den Schaft 2 ist gemäss Figur 2 eine Durchgangsbohrung 6 eingeformt, in welcher ein Schieber 7 aufgenommen ist, der in Richtung des Pfeiles x bewegbar ist.

Einends ist dem Schieber 7 ein Fähnchen 8 aufgesetzt, welches aus dem Schaft 2 bzw. einem Schaftschlitz 9 herausragt. Dieses Fähnchen 8 besitzt zwei Bohrungen 10.1 und 10.2, welche der Aufnahme eines später beschriebenen Halteelements dienen.

Andernends des Fähnchens 8 weist der Schieber 7 eine stirnwärtige Sacklochbohrung 11 auf, in die ein Zapfen 12 eingesetzt ist. Der Zapfen 12 dreht frei in der Sacklochbohrung 11 und ist Bestandteil eines Betätigungselementes 13, mit dem der Schieber 7 in Richtung x bewegt wird. Hierzu weist das Betätigungselement 13 einen Gewindeabschitt 14 mit einem Aussengewinde 15 auf, welches ein nicht näher gezeigtes Innengewinde in der Durchgangsbohrung 6 kämmt.

An den Gewindeabschnitt 14 schliesst ein Stangenabschnitt 16 an, der mit einem Sechskant 17 endet. Wird dieser Sechskant 17 von einem entsprechenden Werkzeug angegriffen, so kann das Betätigungselement 13 um seine Längsachse A gedreht werden, wobei es gegenüber dem Schieber 7 frei dreht. Über das Aussengewinde 15 schraubt sich jedoch das Betätigungselement 13 weiter in die Durchgangsbohrung 6 ein, so dass es den Schieber 7 in Richtung x bewegt, was eine entgegengesetzte Bewegung des Grundkörpers 1 in Richtung x₁ zur Folge hat.

Quer zur Längsachse A durchsetzen den Schaft 2 drei Querbohrungen 18.1, 18.2 und 18.3, welche der Aufnahme von nicht näher gezeigten Befestigungselementen, wie beispielsweise Schrauben, dienen.

Die Funktionsweise der vorliegenden Erfindung ist folgende:

Gemäss den Figuren 3 und 4 wird der Zugapparat R an einen Oberkiefer 19 eines menschlichen Schädels 20 so angelegt, dass der Haken 4 den Oberkiefer 19 hinter dem letzten Backenzahn 21 hintergreift. Nunmehr erfolgt eine Fixierung des Zugapparates R durch Einsetzen von Schrauben 22 in die Querbohrungen 18, wobei diese Schrauben 22 die Querbohrungen 18 durchgreifen und in den Oberkiefer 19 eingeschraubt werden. Das Betätigungselement 13 verläuft dabei etwa parallel zum Oberkiefer 19 bzw. zu den Backenzähnen 21.

Nunmehr wird mit dem Fähnchen 8 die oben erwähnte Halterung 23 verbunden, welche beispielsweise aus einem Drahtstück bestehen kann. Diese Halterung 23 wird andernends des Fähnchens 8 an einer anderen Stelle des Schädels 20, bevorzugt am Backenknochen, festgelegt, wobei diese Festlegung durch ein Titanplättchen 24 geschehen kann.

Wird nun an dem Betätigungselement 13 gedreht, so verschiebt sich das Fähnchen 8 in Richtung x, was gleichzeitig bewirkt, dass der Schaft 2 in entgegengesetzter Richtung x₁ bewegt wird und über den Haken 4 den Oberkiefer 19 nach vorne mitnimmt. Der Druck auf den Oberkiefer 19 bewirkt, dass dieser im Laufe der Zeit nach vorne versetzt wird, so dass es zur Beseitigung beispielsweise eines Überbisses, Gaumenspaltes oder Kreuzbisses kommt. Jeweils nach einer bestimmten Zeit wird an dem Betätigungselement 13 weiter gedreht, so dass im Laufe der Zeit der Oberkiefer 19 diesem Druck nachgibt und nach vorne wandert. Ziel ist schlussendlich eine korrekte Okklusion zwischen Oberkiefer und Unterkiefer.

Sollte es sich als notwendig erweisen, kann in den Oberkiefer 19 etwa parallel zu dem Betätigungselement 13 eine Schwächungslinie eingefräst werden. Dies geschieht allerdings dann nur an denjenigen Stellen, an denen sich der Oberkiefer 19 besonders einem Druck des Zugapparates R entgegenstellt. Normalerweise ist diese Schwächung nicht notwendig.

## Patentansprüche

1. Zugapparat zur Korrektur eines Oberkiefers (19) an einem Schädel (20), beispielsweise zur Beseitigung eines Überbisses, Kreuzbisses oder Gaumenspaltes,
dadurch gekennzeichnet,
dass ein Grundkörper (1) mit Elementen (4, 18, 22) zum Festlegen am Oberkiefer (19) versehen und in Korrekturrichtung (x₁) bewegbar ist.

2. Zugapparat nach Anspruch 1, dadurch gekennzeichnet, dass der Grundkörper (1) aus einem Schaft (2) und einem Haken (4) zum Hintergreifen des Oberkiefers (19) besteht.

3. Zugapparat nach Anspruch 2, dadurch gekennzeichnet, dass der Schaft (2) über einen Bogen (3) mit dem Haken (4) verbunden ist, wobei Bogen (3) und Haken (4) einen Hinterschnitt (5) ausbilden.

4. Zugapparat nach einem der Ansprüche 1 - 3, dadurch gekennzeichnet, dass der Grundkörper (1) bzw. der Schaft (2) von zumindest einer Querbohrung (18) zur Aufnahme von einer Schraube (22) od. dgl. durchsetzt ist.

5. Zugapparat nach wenigstens einem der Ansprüche 1 - 4, dadurch gekennzeichnet, dass in den Grundkörper (1) bzw. den Schaft (2) eine Durchgangsbohrung (6) eingeformt, in dieser ein Schieber (7) eingesetzt und diesem ein Betätigungselement (13) zugeordnet ist.

6. Zugapparat nach Anspruch 5, dadurch gekennzeichnet, dass der Schieber (7) entgegen der Korrekturrichtung (x₁) verschiebbar ist.

7. Zugapparat nach Anspruch 5 oder 6, dadurch gekennzeichnet, dass an dem Schieber (7) ein Fähnchen (8) angeordnet ist und aus dem Schaft (2) herausragt.

8. Zugapparat nach Anspruch 7, dadurch gekennzeichnet, dass das Fähnchen (8) über eine Halterung (23) mit einem anderen Teil des Oberkiefers (19) oder des Schädels (20) verbunden ist.

9. Zugapparat nach Anspruch 8, dadurch gekennzeichnet, dass die Halterung (23) ein Draht, Titanplättchen od. dgl. ist, der andernends mit einem Titanplättchen (24) od.dgl. verbunden ist und dieses an einem anderen Teil des Schädels (20) bzw. des Oberkiefers (19) festliegt.

10. Zugapparat nach wenigstens einem der Ansprüche 5 - 9, dadurch gekennzeichnet, dass das Betätigungselement (13) um seine Längsachse (A) drehbar ist und einen Gewindeabschnitt (14) aufweist, der in der Durchgangsbohrung (6) dreht.

11. Zugapparat nach Anspruch 10, dadurch gekennzeichnet, dass das Betätigungselement (13) anschliessend an den Gewindeabschnit (14) mit einem Zapfen (12) in eine Sacklochbohrung (11) in den Schieber (7) stirnwärtig eingreift und gegenüber dem Schieber (7) frei dreht.

12. Zugapparat nach Anspruch 11, dadurch gekennzeichnet, dass andernends des Zapfens (12) an den Gewindeabschnitt (14) ein Stangenabschnitt (16) mit einem Sechskantkopf (17) anschliesst.
